Europäisches Patentamt

European Patent Office

Office européen des brevets

(19)

(11) Veröffentlichungsnummer: **0 214 341 B1**

## EUROPÄISCHE PATENTSCHRIFT

(45) Veröffentlichungstag der Patentschrift: **04.03.92**

(21) Anmeldenummer: **85115965.7**

(22) Anmeldetag: **13.12.85**

(51) Int. Cl.⁵: **A61K 6/04**, B22F 3/10, A61C 5/10

(54) Verfahren zur Herstellung eines metallischen Zahnersatzes.

(30) Priorität: **11.09.85 DE 3532331**

(43) Veröffentlichungstag der Anmeldung:
**18.03.87 Patentblatt 87/12**

(45) Bekanntmachung des Hinweises auf die
Patenterteilung:
**04.03.92 Patentblatt 92/10**

(84) Benannte Vertragsstaaten:
**AT BE CH DE FR GB IT LI LU NL SE**

(56) Entgegenhaltungen:
EP-A- 0 016 315      EP-A- 0 104 320
DE-A- 1 915 977      DE-A- 3 046 334
DE-U- 8 207 485      FR-A- 2 036 946
FR-A- 2 326 907      US-A- 4 432 795

"Liquid Phase Sintering" von Randall M. German, insbesondere Kapitel 7 (S. 157 bis 179, einschl. der dort zitierten Literaturstellen), und Kapitel 8 (S. 1 bis 29, einschl. der dort zitierten Literaturstellen sowie der zugehörigen Abbildungen 8.1-8.21)

"Perspectives in Powder Metallurgy - Fundamentals, Methods, and Applications", Vol. 1:

"New Methods for the Consolidation of Metal Powders", Plenum Press, New York, 1967, herausgegeben von Henry H. Hausner, Kempton H. Roll, Peter K. Johnson, insbesondere Kapitel 13 "Slip Casting of Metal Powders" von Henry H. Hausner (Adjunct Professor am Polytechnic Institute of Brooklyn und Consulting Engineer, New York), S. 221-338

(73) Patentinhaber: **Degussa Aktiengesellschaft**
**Weissfrauenstrasse 9**
**W-6000 Frankturt am Main 1(DE)**

(72) Erfinder: **Groll, Werner, Dipl.-Ing.**
**Kardinal Döpfner Strasse 5**
**W-8757 Karlstein(DE)**
Erfinder: **Rothaut, Josef, Dr. Dipl.-Phys.**
**247 Harmon Avenue**
**Fort Lee, N.J. 07024(US)**
Erfinder: **Klaus, Angela**
**Feldstrasse 9**
**W-6450 Hanau 8(DE)**
Erfinder: **Steinke, Rudi, Dipl.-Ing.**
**Grünaustrasse 7**
**W-6450 Hanau 9(DE)**

EP 0 214 341 B1

**Beschreibung**

Die Erfindung betrifft ein Verfahren zur sintertechnologischen Herstellung eines mit Keramik oder Kunststoffen verblendbaren Zahnersatzes mit metallischer Gefügematrix aus einer Mischung von Metallpulvern und gegebenenfalls Glas- oder Keramikpulvern, das mit einer Anmischflüssigkeit zu einer aufstreichbaren Masse aufbereitet wird, mit der auf einem als Brennträger dienenden Modell der zu versorgenden Zähne aus Keramik der Zahnersatz mit der bei Dentalkeramiken üblichen Technik modelliert und anschließend auf dem Modell gesintert wird.

Die Herstellung metallischen Zahnersatzes zur prothetischen Versorgung bei Zahnerkrankungen beziehungsweise nach Verlust eines oder mehrerer Zähne, wie z.B. Inlays, mit Keramik oder Kunststoff verblendbare oder nicht verblendete Kronen und Brücken, erfolgt üblicherweise mit dem sogenannten "Wachsausschmelzverfahren", einer Feingußtechnik, die hohe Maßhaltigkeit gewährleistet. Die Vorteile der so hergestellten Kronen und Brücken sind neben der Maßhaltigkeit vor allem in der hohen Festigkeit und der vorhandenen Duktilität zu sehen, die bei größeren Brückenkonstruktionen zur Vermeidung von Gewaltbrüchen bei Überbelastung gewährleistet sein müssen. Dagegen ist das Verfahren selbst sehr zeitraubend, material- und geräteintensiv. Die Notwendigkeit der Verwendung von Angußkanälen und Gußkegel verursacht einen gegenüber dem Gewicht des Gußobjektes deutlich erhöhten Materialeinsatz, der bei mehrmaliger Wiederverwendung zu Veränderungen der Legierungseigenschaften führen kann, und - falls er nicht wiederverwendet wird - als Schrott zurückbleibt. Ein weiterer Nachteil dieser Technik ist, daß bei Fehlern im Gußobjekt eine Reparatur nicht möglich ist, sondern der gesamte Herstellprozeß, beginnen bei der Wachsmodellation, wiederholt werden muß.

Ein anderes Verfahren zur Herstellung von metallkappenverstärkten Jacketkronen wird in der EP-OS 0104320 beschrieben. Ein vorgeformtes, mit Falten versehenes Hütchen aus einer Metallfolie, die vorzugsweise aus mehreren Schichten verschiedener Metalle aufgebaut ist, wird über das Modell des präparierten Zahnstumpfes gelegt und an dieses mit einem geeigneten Werkzeug anrotiert. Bei der Glühung mit einem Bunsenbrenner werden die übereinandergelegten Falten verschweißt und es liegt eine Metallkappe mit einer Wandstärke von ca. 100 $\mu$m vor, die dann mit Dentalkeramik verblendet wird. Der Arbeits- und Apparateaufwand wird gegenüber dem Wachsausschmelzverfahren zwar deutlich verringert, doch erreicht der so gefertigte Zahnersatz bei weitem nicht die Festigkeitseigenschaften eines gegossenen Zahnersatzes, so daß eine Herstellung von Brücken mit diesem Verfahren nicht möglich ist. Zudem erfordert die stets gleiche Dicke der Metallfolie bei stark abgeschliffenen Stümpfen beziehungsweise bei großen Zähnen, speziell bei Molaren, eine sehr dicke Keramikmodellation, so daß die Gefahr des Keramikbruches insbesondere im Seitenzahnbereich sehr groß ist.

Eine bekannte Methode zur Herstellung von vollkeramischen Kronen ist die Jacketkronentechnik, bei der eine aluminiumoxidhaltige Keramikmasse auf eine vorher entsprechend der Form des Zahnstumpfes geformte Pt-Folie aufgebracht und gesintert wird. Die Krone wird frei von Hand modelliert, so daß der gesamte, zur Herstellung von Gußkronen erforderliche Gerätepark nicht benötigt wird. Die Modelliereigenschaften der Keramikmasse erlauben eine exakte Nachbildung der kompliziertesten Zahnformen. Wesentlicher Nachteil dieses Typs von Zahnersatz ist die dem keramischen Material eigene Sprödigkeit, die bei momentaner Überbelastung zum katastrophalen Bruch führt. Die Festigkeit ist ebenfalls nicht ausreichend, um dickwandige Kronen und größere Brücken zu fertigen.

In der DE-OS 19 15 977 wird ein Verfahren zur Herstellung von Zahnersatz beschrieben, das von Metall- beziehungsweise Legierungspulver mit einer Partikelgröße von 2 bis 25 $\mu$m ausgeht, die mit Hilfe eines unterhalb der Sintertemperatur flüchtigen Bindemittels zu einer Paste angeteigt werden. Diese Paste wird auf einem dem Zahnstumpf maßgetreu nachgebildeten Modell, das als Brennträger dient, mit einem Spatel frei modelliert, das Bindemittel bei höherer Temperatur ausgetrieben und die Metallteilchen zusammengesintert. Dieses Verfahren hat jedoch den Nachteil, daß mit der Paste keine hohe Verdichtung der Metallpulvermasse erreichbar ist, so daß es beim Sintern zu einer relativ starken Schrumpfung kommt. Die für einen Zahnersatz geforderte hohe Paßgenauigkeit kann mit diesem Verfahren daher nicht erreicht werden, auch nicht bei Verwendung von sehr feinen, kugelförmigen Pulvern, die andererseits nur mit geringer Ausbeute und deshalb nur unter hohen Kosten herstellbar sind.

Es war Aufgabe der vorliegenden Erfindung, ein Verfahren zur sintertechnologischen Herstellung eines mit Keramik oder Kunststoff verblendbaren Zahnersatzes mit metallischer Gefügematrix zu entwickeln aus einer Mischung von Metallpulvern und gegebenenfalls Glas- oder Keramikpulvern, das mit einer Anmischflüssigkeit zu einer aufstreichbaren Masse aufbereitet wird, mit der auf einem als Brennträger dienenden Modell der zu versorgenden Zähne aus Keramik der Zahnersatz mit der bei Dentalkeramiken üblichen Technik modelliert und anschließend auf dem Modell gesintert wird. Bei diesem Verfahren sollte beim Sintern die Schrumpfung so klein wie möglich gehalten werden, um einen paßgenauen Zahnersatz zu erhalten, der eine hohe Festigkeit besitzt, weitestgehend frei von offenen Poren ist und preisgünstig hergestellt werden kann.

Diese Aufgabe wurde erfindungsgemäß dadurch gelöst, daß die Pulvermischung eine mehrmodale Größenverteilung aus groben und feinen Fraktionen aufweist, wobei für die groben Fraktionen überwiegend Pulver kugeliger Gestalt mit Korngrößen zwischen 30 und 100 $\mu$m und für die feinen Fraktionen Pulver beliebiger Gestalt mit Korngrößen unter 50 $\mu$m verwendet werden, daß diese Pulvermischung mit Wasser in einen Schlicker überführt wird, und daß die Sintertemperatur der Schlickermasse so gewählt wird, daß sie die Solidustemperatur mindestens eines Bestandteils der Pulvermischung übersteigt und im Falle einer vorgesehenen Verblendung mit Keramik mindestens 50° C über der Aufbrenntemperatur der Keramik liegt.

Bevorzugte Ausführungsformen des Verfahrens ergeben sich aus den Unteransprüchen.

Bei dem erfindungsgemäßen Verfahren wird eine Mischung aus Metallpulvern der für die gewünschte Legierungszusammensetzung notwendigen Elemente in den entsprechenden Mengenverhältnissen beziehungsweise Legierungspulver und gegebenenfalls Keramikpulver mit Wasser zu einem Schlicker angemischt, dessen Konsistenz und Modellationseigenschaft denen von üblicher Dental- beziehungsweise Verblendkeramik entsprechen. Zur Erzielung einer möglichst hohen Rohdichte und dementsprechend einer geringen Schrumpfung beim Sintern hat sich die Verwendung von Pulvermischungen mit mehrmodalen Größenverteilungen der eingesetzten Metall- beziehungsweise Keramik-Pulver als wesentlich erwiesen, wobei Pulver mit Korngrößen kleiner 100 $\mu$m Verwendung finden müssen. Der Anteil der Keramikpulver darf nur so groß gewählt werden, daß stets eine metallische Gefügematrix gewährleistet ist. Der angeteigte Schlicker wird dann mit den bei Dentalkeramik üblichen Techniken und Geräten auf ein maßgetreues Modell der zu versorgenden Zähne modelliert und durch geeignete, ebenfalls bei der Herstellung von Keramikzähnen beziehungsweise Keramikverblendungen bekannten Techniken (z.B. Rütteln mit dem Riffelteil eines Modellierinstrumentes) verdichtet. Während des Verdichtungsprozesses wird Flüssigkeit ausgetrieben, wodurch sich die Pulverteilchen in günstigere Positionen umordnen und näher zusammenrücken können. Das verwendete Modell, das aus einer feuerfesten Keramik besteht, wird vorteilhafterweise entsprechend der bekannten Schrumpfung der Legierung vor dem Modellieren mit geeigneten Werkstoffen vergrößert und gegen zu starke Flüssigkeitsaufnahme isoliert.

Durch diese Maßnahmen wird eine optimale Rohdichte der Schlickermasse und eine geringe Schrumpfung beim Sintern gewährleistet. Zur Erzielung einer hohen Sinterdichte muß ein Sinterverfahren angewendet werden, bei dem die Sintertemperatur über der Solidustemperatur mindestens eines Bestandteiles der Pulvermischung liegt, wobei darauf geachtet werden muß, daß bei einer vorgesehenen Keramikverblendung die Sintertemperatur mindestens 50°C über der Brenntemperatur der Keramik liegt. Die letztere Bedingung muß erfüllt sein, um eine Verformung des metallischen Gerüsts beim Keramikbrand zu vermeiden. Je nach Legierungszusammensetzung wird das Sintern an Luft (z.B. Edelmetalle), unter Schutzgas oder Vakuum durchgeführt. Nach dem Sintern liegt ein hinreichend dichter Zahnersatz mit metallischer Gefügematrix vor.

Mehrmodal bedeutet, daß Pulvermischungen verwendet werden, die eine Größenverteilung mit mehreren Maxima bei unterschiedlichen Partikelgrößen aufweisen.

Zur Erzielung einer hohen Rohdichte der Schlickermasse sind solche Pulvermischungen besonders geeignet, deren grobe Fraktionen im Bereich zwischen 30 $\mu$m und 100 $\mu$m liegen, mit einem Volumenanteil von 30 bis 90 % an der gesamten Pulvermischung, und die vorwiegend kugelige Form aufweisen. Die Form der feinen Pulver ( < 50 $\mu$m) ist an sich beliebig, bevorzugt werden jedoch ebenfalls kugelige beziehungsweise plättchenförmige Pulver.

Vorzugsweise werden die Pulverkomponenten, deren Solidustemperatur größer ist als die Sintertemperatur der Schlickermasse als grobe Fraktionen zugesetzt, während die Pulverkomponenten, deren Solidustemperatur kleiner ist als die Sintertemperatur der Schlickermasse als Feinanteil zugegeben werden. Setzt man nämlich die höherschmelzende Komponente als Feinanteil zu, so kann es zur Bildung eines starren Gerüstes durch ein Zusammensintern der Pulverpartikel beim Trocknen beziehungsweise beim Aufheizen auf Brenntemperatur kommen. Eine Verdichtung durch Teilchenumlagerung beim Flüssigphasensintern kann dann nicht mehr erreicht werden. Die flüssige Phase, die beim Überschreiten der Liquidustemperatur der niedrigschmelzenden Pulverkomponente entsteht, dringt in das poröse Gerüst der hochschmelzenden Komponente ein, so daß die vorher von diesem besetzten Stellen als Poren zurückbleiben.

Vorteilhafte Sintertemperaturen für die sintertechnologische Herstellung von Zahnersatz sind Temperaturen im Bereich zwischen der Solidus-Temperatur der gesinterten Legierung $T_{Solidus}$ und $T_{Solidus}$ minus 200°C, wobei die Randbedingungen, daß mindestens eine Pulverkomponente eine Solidustemperatur kleiner der Sintertemperatur besitzen muß und daß die Sintertemperatur im Falle einer Keramikverblendung 50°C oberhalb der Aufbrenntemperatur der Keramik liegen muß, berücksichtigt werden müssen. Die flüssige Phase kann während des Sinterprozesses aufgrund stattfindender Legierungsbildung ganz oder teilweise aufgebraucht werden. Die Anwendung der beschriebenen Sintertemperaturen im Bereich zwischen $T_{Solidus}$

3

und $T_{Solidus}$ minus 200ºC setzt voraus, daß die Pulvermischung aus mindestens zwei Metallbeziehungsweise Legierungspulvern mit unterschiedlicher Solidustemperatur besteht.

Bei Pulvermischungen, deren unterschiedliche Fraktionen aus nur einer Legierung bestehen, kann vorteilhafterweise eine Sintertemperatur zwischen $T_{Solidus}$ und $T_{Liquidus}$ angewendet werden. Ein Teil der Legierung liegt dann - entsprechend der Phasenbeziehung fest/flüssig - als schmelzflüssige Phase vor. Die flüssige Phase darf dabei nur in solchem Ausmaß auftreten, daß die Formstabilität des Sinterkörpers während des Sinterns erhalten bleibt.

Zur Herstellung von Brücken können Fertigteile, wie z.B. Drähte, Profile oder Metallzähne, verwendet werden, die bei der Modellation der Kronenkappen mit eingeschlickert werden und dort festsintern beziehungsweise an die gesinterten Formkappen angelötet werden. Durch diese Maßnahme wird eine bessere Paßgenauigkeit erreicht, da dann die Zwischenglieder beim Sintern nicht schrumpfen. Eine weitere Möglichkeit zur Herstellung von Brücken besteht darin, die einzelnen Zähne - auch die Zwischenglieder - mit dem erfindungsgemäßen Verfahren herzustellen und diese dann zu verlöten.

Zur Verbesserung der Paßgenauigkeit des Zahnersatzes kann der Modellstumpf mit einem rückstandslos verbrennenden Werkstoff, wie z.B. Wachs, überzogen werden, wobei die Dicke des Überzuges so gewählt wird, daß die Umfangsvergrößerung der zu erwartenden Schrumpfung der Schlickermasse beim Sintern entspricht. Auf diesen Überzug wird der Schlicker aufgetragen und verdichtet. Der rückstandslos verbrennende Werkstoff wird dann bei geeigneter Temperatur ausgebrannt. Beim Sintern schrumpft der Formkörper (z.B. eine Krone) auf den Stumpf, so daß dessen Form genau nachgebildet wird.

Außerdem kann der Brennträger vor dem Auftragen des Schlickers mit einem Metall überzogen werden, dessen Schmelzpunkt höher liegt als der der zu sinternden Legierung. Auf diesen so vorbereiteten Modellstumpf wird dann der Schlicker aufgetragen und durch Flüssigphasensintern verdichtet. Die flüssige Phase benetzt den metallisierten Modellstumpf und gewährleistet, daß sich die Legierung auch im Bereich des Keramikstumpfes an die Form anschmiegt.

Zur Herstellung der Schlicker werden die Metallpulvermischungen mit Wasser versetzt, das vorzugsweise Elektrolyte enthält, wie z.B. Soda, Natriumhydroxid oder Strontiumchlorid. Auch können dem Wasser ein- oder mehrwertige Alkohole zugesetzt werden.

Für die Herstellung eines paßgenauen Zahnersatzes auf sintertechnologischem Weg ist eine möglichst hohe Gründichte der Schlickermasse vor dem Sintern wichtig, um die Sinterschrumpfung zu minimieren. Dies erreicht man dadurch, daß Pulvermischungen aus einem oder mehreren Metallen beziehungsweise Metallegierungen mit bi- oder mehrmodaler Größenverteilung verwendet werden, wobei sowohl kugelige, plättchenförmige oder anders geformte Partikel verwendet werden können. In Tabelle 1 sind einige Beispiele von Pulverkombinationen aufgeführt. Als Modellpulver wurden Gold-, Platin-und Palladiumpulver in verschiedener Größenverteilung und Teilchenform verwendet. Reine kugelige Pulver (Werkstoff 1) ergeben eine höhere Gründichte als reine plättchenförmige Pulver (Werkstoff 6). Beide Werkstoffe liegen jedoch außerhalb der Erfindung. Durch Zugabe weiterer Pulver kleinerer Partikelgröße zu mehrmodalen Pulvermischungen ist eine deutliche Zunahme der Gründichte erzielbar. Die besten Ergebnisse liefern Schlickermassen, deren grobe Fraktion aus kugelförmigen Teilchen besteht (Werkstoff 2 - 5).

4

Tab. 1

Legend: □ Plättchen   o kugelig   | nadelig

| Werkstoff | Pulverform/-größe (μm) | | | | Zusammensetzung in Gew.-% P1 : P2 : P3 : P4 | rel.Gründichte/% |
|---|---|---|---|---|---|---|
| | P1 | P2 | P3 | P4 | | |
| 1 | o 90-71 | - | - | - | 100/-/-/- | 35 |
| 2 | o 90-71 | o <10 | - | - | 70/30/-/- | 58 |
| 3 | o 90-71 | o <10 | TiO2(<1) | - | 68.42/29.33/2.25/- | 60 |
| 4 | o 90-71 | o <10 | Bi2O3(<2) | - | 66.94/28.68/4.38/- | 73 |
| 5 | o 90-71 | □ <25 | - | - | 90/10/-/- | 56 |
| 6 | □ <50 | - | - | - | 100/-/-/- | 9.8 |
| 7 | □ <50 | o <10 | - | - | 90/10/-/- | 19 |
| 8 | □ <50 | □ <15 (Pt) | □ <15 (Pd) | - | 50/35/15/- | 25 |
| 9 | □ <50 | □ <15 (Pt) | □ <15 (Pd) | TiO2(<1) | 48.74/34.13/14.63/2.5 | 47 |

Für die erreichbare Dichte und Festigkeit sind neben der Größenverteilung, der Größe und der Form der verwendeten Pulver auch die Sintertemperatur von entscheidender Bedeutung. In Tabelle 2 sind die Eigenschaften der gesinterten Legierung Au50 Pt35 Pd15 bei Verwendung verschiedener Ausgangspulvermischungen mit mehrmodaler Größenverteilung nach dem Sintern zusammengestellt. Die erreichten Dichtewerte und 0,2%-Dehngrenzen sind für den Einsatz als Kronen- und Brückenwerkstoffe günstig. Die 0,2-Dehngrenzen von herkömmlichen, gegossenen Dental- und Brückenlegierungen liegen ebenfalls im Bereich

5

von 450 bis 600 MPa. Die so hergestellten Legierungen zeigen eine geschlossene Porosität, was zur Vermeidung von Plaque-Anlagerungen und Korrosionsangriffspunkten wichtig ist. Bei den Legierungen 5 und 7 wurden Dreipunkt-Biegeversuche an 35 mm langen Prüfkörpern mit einem Querschnitt von 3 x 3 mm$^2$ durchgeführt, wobei die im Biegeversuch erzielten Dehngrenzen denen der Druckversuche entsprechen. Die gesinterten Legierungen weisen also auch gegenüber Zugspannungen eine genügend hohe Festigkeit auf. Die deutlich höhere Biegebruchfestigkeit $R_m$ bestätigt, daß eine plastische Verformung vor dem Bruch stattfindet.

Zur Herstellung von Prüfkörpern wurden auch unedelmetallhaltige Legierungen verwendet. Dazu wurde z.B. verdüstes Pulver eine Au-Sn-In-Legierung mit Au und Pt-Pulver gemischt und bei 990°C gesintert. Um eine Oxidation von Sn und In zu verhindern, wurde der zu sinternde Probekörper in eine Graphitbox auf eine Keramikunterlage gestellt und in dieser Box in einem herkömmlichen Keramik-Aufbrennofen gesintert.

| Nr. | Pulverform/-größe um 50% Au 15% Pd 35% Pt | Keramikzusatz Menge / Vol% | Ts / °C | ϱ / % | Rp 0.2 in MPa (Druck) | Biegeversuch Rp/MPa | Biegeversuch Rm/MPa |
|---|---|---|---|---|---|---|---|
| 1 | □ <15  □ <15  □ <25 | | 1200 | 91,8 | 582 +-43 | | |
| 2 | □ <15  □ <15  □ <25 | + 10 Vol% $TiO_2$ | 1300 | 92,7 | 700 +-8 | | |
| 3 | □ <15  □ <15  □ <25 | + 20 Vol% $TiO_2$ | 1200 | 92,0 | 631 +-21 | | |
| 4 | □ <15  □ <15  □ <25 | + 10 Vol% $TiO_2$ | 1200 | 91,9 | 656 +-16 | | |
| 5 | □ <15  □ <15  □ <25 | | 1300 | 93,7 | 710 +-15 | 640 +-30 | 900 +-50 |
| 6 | □ <15  □ <15  □ <25 | + 10 Vol% $Bi_2O_3$ | 1200 | 92,3 | 610 +-20 | | |
| 7 | □ <15  □ <15  □ <50 | | 1300 | 91,8 | 630 +-15 | 650 +-50 | 864 +-100 |
| 8 | □ <15  □ <15  □ <50 | + 10 Vol% $TiO_2$ | 1300 | 97,2 | 720 +-20 | | |
| 9 | o <10  o <10  □ <25 | | 1300 | 91,0 | 566 +-5 | | |

o kugelig  □ plättchenförmig

Tab. 2

Folgende Beispiele sollen das erfindungsgemäße Verfahren näher erläutern:

1. Von einem Meistermodell wird ein Duplikat aus einer hochtemperaturfesten, gießbaren Keramik hergestellt, das später als Brennträger verwendet wird. Auf den Modellstumpf aus der hochtemperaturbeständigen Keramik wird ein Wachskäppchen modelliert, dessen Wandstärke ca. 0,3 mm beträgt. Das Wachs erfüllt zum einen die Funktion der Isolation gegenüber dem Modellstumpf und dient zum anderen

7

als Vergrößerung des Modellstumpfes zum Kompensation der beim Sintern auftretenden Schrumpfung. Das Wachskäppchen wird aus einer Wachsplatte (Dicke 0,3 mm) geformt oder unter Verwendung eines Tauchwachses hergestellt.

Auf den so vorbereiteten Modellstumpf wird ein Schlicker aufgetragen, der 10 Vol.% $TiO_2$ und 90 Vol.% einer Metallpulvermischung enthält. Letztere besteht aus 74,4 Gew.% Au-Pulver (kugelig)< 90 $\mu$m, 18,6 Gew.% Au-Pulver (plättchenförmig) < 10 $\mu$m und 7 Gew.% Pt-Pulver (plättchenförmig) < 15 $\mu$m. Als Anmischflüssigkeit wird Wasser mit 0,5 g/l Strontiumchlorid verwendet. Dieser Schlicker hat Eigenschaften, die denen von Dentalkeramikschlickern entsprechen. Mit der beim Modellieren mit Dentalkeramik üblichen Technik und den dazu verwendeten Instrumenten (Pinsel, Spatel, Riffelteil etc.) wird mit dem Schlicker eine verblendbare Kronenkappe aufgebaut. Nach Beendigung der Modellation wird die gesamte Anordnung in einem Auswachsofen bei 200°C 30 Minuten gehalten. Während dieser Zeit brennt das Wachs rückstandsfrei aus. Danach wird die entwachste Anordnung zunächst in die Trockenkammer eines Keramik-Aufbrennofens gegeben und bei 600°C 15 Minuten getrocknet, anschließend in die auf 1200°C vorgeheizte Brennkammer umgesetzt und dort 15 Minuten gesintert. Nach dem Sintern kühlt die Kronenkappe an Luft ab und kann dann vom Brennträger abgenommen werden. Die Keramikverblendung wird ohne Zwischenbehandlung direkt auf die gesinterte Kappe in bekannter Weise aufgebracht. Die so hergestellte Krone besitzt eine metallische Matrix und eine hohe Paßgenauigkeit, verbunden mit einer hohen Festigkeit.

2. Die Herstellung und Vorbereitung des Modellstumpfes erfolgt entsprechend Beispiel 1. Der mit Wasser angeteigte Schlicker besteht wiederum aus 10 Vol.% $TiO_2$ und 90 Vol.% einer Metallpulvermischung, die sich ihrerseits aus 65,1 Gew.% Au-Pulver (kugelig) der Fraktion 36 - 25 $\mu$m, 27,9 Gew.% Au-Pulver (plättchenförmig) <25 $\mu$m und 7 Gew.% Pt-Pulver (plättchenförmig)<15 $\mu$m zusammensetzt. Mit diesem Schlicker wird eine Krone mit Bißfläche unter Verwendung der bei Keramikverblendung üblichen Technik modelliert. Aufgrund der hervorragenden Modellierbarkeit des Schlickers lassen sich Feinheiten der Okklusalflächen ausarbeiten. Die Arbeitsschritte Entwachsen, Trocknen und Sintern verlaufen entsprechend Beispiel 1. Nach dem Sintern wird die Krone von dem Brennträger abgenommen, die Oberfläche feingeschliffen und anschließend poliert. Auch diese Krone besitzt eine hohe Paßgenauigkeit. Poren sind nicht erkennbar.

**Patentansprüche**

1. Verfahren zur sintertechnologischen Herstellung eines mit Keramik oder Kunststoff verblendbaren Zahnersatzes mit metallischer Gefügematrix aus einer Mischung von Metallpulvern und gegebenenfalls Glas- oder Keramikpulvern, das mit einer Anmischflüssigkeit zu einer aufstreichbaren Masse aufbereitet wird, mit der auf einem als Brennträger dienenden Modell der zu versorgenden Zähne aus Keramik der Zahnersatz mit der bei Dentalkeramiken üblichen Technik modelliert und anschließend auf dem Modell gesintert wird,
   dadurch gekennzeichnet,
   daß die Pulvermischung eine mehrmodale Größenverteilung aus groben und feinen Fraktionen aufweist, wobei für die groben Fraktionen überwiegend Pulver kugeliger Gestalt mit Korngrößen zwischen 30 und 100 $\mu$m und für die feinen Fraktionen Pulver beliebiger Gestalt mit Korngrößen unter 50 $\mu$m verwendet werden, daß diese Pulvermischung mit Wasser in einen Schlicker überführt wird und daß die Sintertemperatur der Schlickermasse so gewählt wird, daß sie die Solidustemperatur mindestens eines Bestandteiles der Pulvermischung übersteigt und im Falle einer vorgesehenen Verblendung mit Keramik mindestens 50° C über der Aufbrenntemperatur der Keramik liegt.

2. Verfahren zur Herstellung von Zahnersatz nach Anspruch 1, dadurch gekennzeichnet, daß der Volumenanteil der groben Fraktionen 30 bis 90 % beträgt.

3. Verfahren zur Herstellung von Zahnersatz nach Anspruch 1 und 2, dadurch gekennzeichnet, daß diejenigen Pulverkomponenten, deren Solidustemperatur unterhalb der Sintertemperatur der Schlickermasse liegen, als feine Fraktionen zugegeben werden.

4. Verfahren zur Herstellung von Zahnersatz nach Anspruch 1 bis 3, dadurch gekennzeichnet, daß bei Schlickermassen mit Pulvern mehrerer Metalle beziehungsweise Legierungen die Sintertemperatur im Bereich zwischen ($T_{solidus}$ minus 200° C) und $T_{solidus}$ der gesinterten Metallmatrix liegt, jedoch über der Solidustemperatur einer oder mehrerer Pulverkomponenten.

**5.** Verfahren zur Herstellung von Zahnersatz nach Anspruch 1 bis 4, dadurch gekennzeichnet, daß bei Schlickermassen mit Pulvern nur einer Legierung die Sintertemperatur zwischen der Solidus- und der Liquidustemperatur der Metallmatrix liegt.

**6.** Verfahren zur Herstellung von Zahnersatz nach Anspruch 1 bis 5, dadurch gekennzeichnet, daß auf die Oberfläche der gesinterten Schlickermasse ein niedrigschmelzendes Metall beziehungsweise Metall-Legierung zur Veredlung der Oberfläche und zur Schließung von Poren aufgesintert wird.

**7.** Verfahren zur Herstellung von Zahnersatz nach Anspruch 1 bis 6, dadurch gekennzeichnet, daß auf dem als Brennträger dienenden Modell entsprechend der Schrumpfung beim Sintern eine Schicht aus einem rückstandslos verbrennenden Werkstoff aufgebracht wird.

**Claims**

**1.** A process based on sintering technology for the production of false teeth faceable with ceramics or plastics and comprising a metallic structural matrix of a mixture of metal powders and optionally glass or ceramic powders which is made up with a mixing liquid into a spreadable paste with which the false teeth are modelled by the technique normally applied in dental ceramics on a model of the teeth to be supplied serving as the firing support and are subsequently sintered on the model, characterized in that the powder mixture has a multimodal size distribution of coarse and fine fractions, spherical-particle powders having particle sizes of 30 to 100 $\mu$m mainly being used for the coarse fractions and powders of any particle shape with particle sizes below 50 $\mu$m being used for the fine fractions, in that the powder mixture is converted with water into a slip and in that the sintering temperature of the slip is selected so that it exceeds the solidus temperature of at least part of the powder mixture and, where the false teeth are to be faced with ceramic, is at least 50$^{\circ}$C above the firing-on temperature of the ceramic.

**2.** A process for the production of false teeth as claimed in claim 1, characterized in that the coarse fractions make up 30 to 90% by volume.

**3.** A process for the production of false teeth as claimed in claims 1 and 2, characterized in that those powder components of which the solidus temperature is below the sintering temperature of the slip are added as fine fractions.

**4.** A process for the production of false teeth as claimed in claims 1 to 3, characterized in that, in the case of slips containing powders of several metals or alloys, the sintering temperature is in the range from ($T_{solidus}$ minus 200$^{\circ}$C) and $T_{solidus}$ of the sintered metal matrix, but is above the solidus temperature of one or more powder components.

**5.** A process for the production of false teeth as claimed in claims 1 to 4, characterized in that, in the case of slips containing powders of only one alloy, the sintering temperature is between the solidus temperature and the liquidus temperature of the metal matrix.

**6.** A process for the production of false teeth as claimed in claims 1 to 5, characterized in that a low-melting metal or metal alloy is sintered onto the surface of the sintered slip to finish the surface and to close pores.

**7.** A process for the production of false teeth as claimed in claims 1 to 6, characterized in that a layer of a completely combustible material is applied to the model serving as firing support in accordance with the shrinkage occurring during sintering.

**Revendications**

**1.** Procédé pour la préparation en technique de frittage d'un produit de remplacement des dents recouvert de céramique ou de matière synthétique, avec une matrice de structure métallique constituée à partir d'un mélange de poudres métalliques et éventuellement de poudres de verre ou de céramique, procédé préparé avec un liquide de conditionnement en une masse pouvant être étendue, avec laquelle le produit de remplacement des dents est modelé en technique usuelle pour la céramique

**EP 0 214 341 B1**

dentaire sur un modèle des dents à faire, servant de support de cuisson et ensuite est fritté sur le modèle, caractérisé en ce que le mélange de poudres comprend une répartition granulométrique plurimodale de fractions grossières et fines, dans laquelle on utilise pour les fractions grossières principalement de la poudre de constitution sphérique avec des grandeurs de grains, entre 30 et 100 x $10^{-6}$ m et pour les fractions fines de la poudre de structure quelconque et de grandeur de grains inférieure à 50 x $10^{-6}$ m, en ce que le mélange de poudre est transformé avec de l'eau en une bouillie et, en ce que la température de frittage de la masse de bouillie est choisie de façon qu'elle dépasse la température du solidus au moins d'un composant du mélange de poudre et dans le cas d'un revêtement prévu en céramique qu'elle se situe au moins à 50°C au-dessus de la température de cuisson de la céramique.

2. Procédé de préparation de produit de remplacement de dents selon la revendication 1, caractérisé en ce que la quote-part en volume des fractions grossières atteint 30 à 90 %.

3. Procédé de préparation de produit de remplacement de dents selon les revendications 1 et 2, caractérisé en ce que ces composants pulvérulents, dont la température de solidus se situe en-dessous de la température de frittage de la masse de bouillie, sont ajoutés comme fraction fine.

4. Procédé de préparation de produit de remplacement de dents selon les revendications 1 à 3, caractérisé en ce que pour des masses de bouillie confectionnées avec des poudres de plusieurs métaux ou alliages, la température de frittage se situe dans la zone entre $T_{solidus}$ moins 200°C et $T_{solidus}$ de la matrice métallique frittée, et toutefois au-dessus de la température du solidus d'un ou de plusieurs composants pulvérulents.

5. Procédé de préparation de produit de remplacement de dents selon les revendications 1 à 4, caractérisé en ce que pour des masses de bouillie provenant de poudres d'un seul alliage la température de frittage se situe entre la température du solidus et la température du liquidus de la matrice métallique.

6. Procédé de préparation de produit de remplacement de dents selon les revendications 1 à 5, caractérisé en ce que sur la surface de la masse de bouillie frittée, on opère le frittage d'un métal à bas point de fusion ou d'un alliage métallique pour l'amélioration de la surface et pour fermer les pores.

7. Procédé de préparation de produit de remplacement de dents selon les revendications 1 à 6, caractérisé en ce que sur le modèle servant de support de cuisson est appliquée une couche d'une matière combustible sans résidu correspondant au retrait pendant le frittage.